## Europäisches Patentamt
## European Patent Office
## Office européen des brevets

(19)

(11) Veröffentlichungsnummer: **0 160 260**
**A2**

(12) # EUROPÄISCHE PATENTANMELDUNG

(21) Anmeldenummer: 85104864.5

(22) Anmeldetag: 22.04.85

(51) Int. Cl.⁴: **C 12 N 11/04**

(30) Priorität: 02.05.84 DE 3416141
02.05.84 DE 3416142
28.07.84 DE 3427888
28.07.84 DE 3427890

(43) Veröffentlichungstag der Anmeldung:
06.11.85 Patentblatt 85/45

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Egerer, Peter, Dr.
Claudiusweg 7
D-5600 Wuppertal 1(DE)

(72) Erfinder: Haese, Wilfried, Dr.
Hauweg 20
D-4050 Mönchengladbach-Neuwerk(DE)

(72) Erfinder: Perrey, Hermann, Dr.
Auf der Rheinaue 8
D-4150 Krefeld 11(DE)

(72) Erfinder: Schmidt-Kastner, Günther, Prof.-Dr.
Falkenweg 59
D-5600 Wuppertal 1(DE)

(54) Verfahren zur Immobilisierung von biologischem Material.

(57) Die Erfindung betrifft Verfahren zur Immobilisierung von biologischem Material durch Einschluß in polymerisierte Verbindungen, gegebenenfalls in Perlform, das nach diesem Verfahren erhältliche immobilisierte biologische Material und dessen Verwendung zu Biotransformationen.

Sie betrifft ebenfalls Verfahren zur Immobilisierung von Protaminobacter rubrum durch Einschluß in polymerisierte Verbindungen, das nach diesen Verfahren erhältliche Präparat und dessen Verwendung zu Herstellung von Isomaltulose aus Sucrose.

EP 0 160 260 A2

BAYER AKTIENGESELLSCHAFT      5090 Leverkusen, Bayerwerk
Konzernverwaltung RP
Patentabteilung              Bu/m-c

## Verfahren zur Immobilisierung von biologischem Material

Die Erfindung betrifft Verfahren zur Immobilisierung von biologischem Material durch Einschluß in polymerisierte Verbindungen, gegebenenfalls in Perlform, das nach diesem Verfahren erhältliche immobilisierte biologische Material und dessen Verwendung zu Biotransformationen.

Die Immobilisierung von Biokatalysatoren führt in der Regel zu verfahrenstechnischen und wirtschaftlichen Vorteilen. Es lassen sich chemische und physikalische Eigenschaften, manchmal auch Selektivität und Spezifität des Biokatalysators durch die Immobilisierung modifizieren.

Die Methoden zur Immobiliserung lassen sich allgemein in folgende Gruppen einteilen

Le A 22 947-Ausland

- Adsorption an vorgefertigte Träger
- Kovalente Bindung an Träger
- Quervernetzung
- Einschluß in eine Polymermatrix.

Das Einschlußverfahren beruht darauf, daß das Biomaterial hinsichtlich seines Bewegungsraumes während der Polymerisation in ein Netzwerk mehr oder minder einheitlicher Maschen- und Porenweite eingeschlossen wird. Dieses Polymernetzwerk sollte für den Biokatalysator unpassierbar sein, während es dem Substrat und dem Produkt einer an diesem Katalysator durchgeführten Umsetzung praktisch keinen Widerstand bieten sollte.

Herkömmliche Verfahren verwenden niedermolekulare, hydrophile Monomere zum Aufbau des Netzwerkes, beispielsweise Hydroxyethylacrylat, Hydroxyethylmethacrylat, Hydroxypropylacrylat, Hydroxypropylmethacrylat, Acrylamid u.a., die mit der wäßrigen Lösung oder Dispersion des Biomaterials gemischt und anschließend polymerisiert werden.

Zum Beispiel werden in der US-PS 3 788 950 monofunktionelle Acrylsäurederivate wie Acrylamid mit einer geringen Menge an Vernetzer wie N,N-Methylenbisacrylamid verwendet. In der US-PS 3 859 169 werden z.B. Monoester der Acrylsäure mit Glykolen in Verbindung mit einem Ver-

Le A 22 947

netzer und einem löslichen Polymer verwendet. Die US-PS
3 860 490 beschreibt den Einsatz niedermolekularer Hydroxyalkyl-acrylate und -methacrylate zur Bildung des
Polymernetzwerkes.

Bei fast allen Verfahren treten jedoch Schwierigkeiten
bei der Steuerung und Reproduzierbarkeit der Permeabilitäten für das biologische Material sowie für dessen
Substrate und Produkte auf. Für eine Eignung als Immobilisierungsmaterial sind diese Größen jedoch essentiell.
Außerdem ist die Verwendung niedermolekularer Acrylsäurederivate wegen ihrer Toxizität nachteilig. Es kann während der Polymerisation zu einer Schädigung des Biokatalysators kommen, oder im Polymerisat befindliche Restmonomere schränkten die Verwendung des immobilisierten
Biokatalysators zur Herstellung von Produkten für die
Nahrungsmittelindustrie oder für die pharmazeutische
Industrie stark ein.

Bei fast allen diesen Verfahren können jedoch nur durch
äußere Formgebung Materialien wie Filme, Folien, Gußteile u.a. erhalten werden. Zur Verwendung dieser Materialien ist oftmals ein nachträgliches Bearbeiten
notwendig, womit sich jedoch die für Katalysatoren
günstige Kugelform kaum erreichen läßt.

Die US-PS 3 860 490 erwähnt die Möglichkeit der Herstellung von Perlen durch Suspensionspolymerisation in
einem inerten Medium, jedoch durch die Verwendung niedermolekularer Hydroxy-alkylacrylate und -methacrylate

Le A 22 947

bestehen bei diesem Verfahren die gleichen Nachteile und Schwierigkeiten die oben erwähnt wurden.

Es wurde nun ein Verfahren zur Immobilisierung von biologischem Material durch Einschluß in polymerisierbare Verbindungen gefunden, das im wesentlichen darin besteht, gut wasserlösliche höhermolekulare polymerisierbare Verbindungen als polymerisierbares Material zu verwenden, das zwei oder mehr polymerisierbare funktionelle Gruppen pro Molekül und ein Molekulargewicht über 400 besitzt.

Die Erfindung betrifft demnach ein Verfahren zur Immobilisierung von biologischem Material durch Einschluß in polymerisierbare Verbindungen, das dadurch gekennzeichnet ist, daß wäßrige Lösungen oder Dispersionen des biologischen Materials mit einer wäßrigen Lösung von gut wasserlöslichen, höhermolekularen, polymerisierbaren Verbindungen, die zwei oder mehr polymerisierbare funktionelle Gruppen pro Molekül und ein Molekulargewicht über 400 besitzen, gemischt werden und die Mischung polymerisiert wird.

Durch die Verwendung der erfindungsgemäß verwendeten höhermolekularen, bevorzugt zwei oder mehr, ungesättigte Gruppen enthaltenden, härtbaren, wasserlöslichen Verbindungen läßt sich eine bessere Steuerung und Reproduzierbarkeit der Permeabilitäten erreichen, da sich diese Eigenschaften im wesentlichen durch die höhermolekularen, wasserlöslichen Verbindungen ergeben. Außerdem besteht durch die Verwendung von insbesondere höhermole-

kularen Verbindungen kein Toxizitätsproblem. Niedermolekulare toxische Verunreinigungen können außerdem vor dem Mischen mit dem biologischen Material entfernt werden.

Desweiteren wurde gefunden, daß die gut wasserlöslichen erfindungsgemäßen Verbindungen zunächst in einer gewissen Menge Wasser, Pufferlösung, Salzlösung und ähnlichem aufgelöst werden können, womit diese Polymerlösung der wäßrigen Lösung oder Dispersion des biologischen Materials hinsichtlich bestimmter Eigenschaften wie pH-Wert, Salzkonzentration, Viskosität u.a. angepaßt werden kann. Anschließend werden beide Lösungen bzw. die wäßrige Polymerlösung und die Zelldispersion gemischt und polymerisiert. Dieses Verfahren besitzt besonders Vorteile für empfindliche Biokatalysatoren, die dadurch keinen abrupten Änderungen des umgebenden Mediums ausgesetzt werden, wodurch eine Aktivitätseinbuße bei der Herstellung der polymerisierbaren Mischung weitgehend vermieden wird.

Ein weiterer Vorteil der vorliegenden Erfindung ist die mit der guten Wasserlöslichkeit einhergehende große Aufnahmekapazität des Polymeren für wäßrige Lösungen und Suspensionen. Es sind Gewichtsverhältnisse von wäßrigem biologischem Material zum Polymer zwischen 1:1 und 30:1 möglich, besonders geeignet sind Verhältnisse zwischen 4:1 und 20:1.

Durch den möglichen großen Wassergehalt des immobilisierten biologischen Materials vor und nach der Polymerisation befinden sich die Biomaterialien in einer

Le A 22 947

besonders geeigneten, schonenden Umgebung, wodurch die Aktivität stabil gehalten werden kann und wodurch zusätzlich der Substrat- und Produktfluß begünstigt sind. Durch Variation der Struktur und des Molekulargewichts der polymerisierbaren, gut wasserlöslichen, höhermolekularen Verbindungen sind die Eigenschaften des polymeren Netzwerkes in weitem Maße variationsfähig. Dadurch lassen sich die Vernetzungsdichte, die Permeabilität, das Quellverhalten und weitere Eigenschaften optimieren, um den Anforderungen des biologischen Materials zu entsprechen.

Das immobilisierte Biomaterial kann während der Polymerisation durch Formgebung nahezu beliebige Gestalt annehmen. Es sind Folien, Filme, Formteile usw. darstellbar. Weiterhin können durch Einbau von Stützgeweben die mechanischen Eigenschaften modifiziert werden. Ebenso sind Beschichtungen von Elektroden, Membranen, oder anderen Materialien durchführbar.

Weiterhin wurde ein Verfahren zur Herstellung von immobilisiertem biologischem Material durch Einschluß in polymerisierte Verbindungen gefunden, das ladurch gekennzeichnet ist, daß eine wäßrige Lösun; oder Dispersion eine biologischen Materials mit ein: wäßrigen Lösung von gut wasserlöslichen höhermolekul en polymerisierbaren Verbindungen, die zwei oder me i: polymerisierbare funktionelle Gruppen pro Molekül un: Molekulargewicht von über 400 besitzen, gemischt wir und diese Mischung in einem inerten flüssigen Medium z Perlen zerteilt und polymerisiert wird. Als Inertphase t nen insbesondere mit Wasser nicht mischbare Stoffe w e z.B. aliphatische,

Le A 22 947

cycloaliphatische und aromatische Kohlenwasserstoffe wie z.B. Hexan, Cyclohexan, Toluol sowie auch halogenierte Derivate derselben, des weiteren Alkohole, Ether, Ester oder sonstige, nicht wassermischbare Stoffe wie auch Siliconöle und Paraffinöle dienen, bzw. auch Gemische derselben untereinander.

Die durch dieses Verfahren mögliche Herstellung von Biokatalysatoren in Perlform besitzt besondere Vorteile bei der Verwendung des immobilisierten Biomaterials. Einerseits eignet sich die Kugelform besonders zur Füllung von Säulenreaktoren, wobei eine gute Durchströmung gewährleistet ist, Verstopfungen vermieden werden können und gleichzeitig durch die große Oberfläche eine gute Reaktionsgeschwindigkeit für die Umsetzung am Biokatalysator resultiert. Auch in strömenden Systemen ist die Kugelform auf Grund der hydrodynamischen Eigenschaften besonders geeignet.

Ferner stellt das vorliegende Verfahren eine Vereinfachung und Verbesserung der bisherigen Verfahren dar. Die Kugelform wird direkt bei der Polymerisation erhalten und ist durch die Verfahrensbedingungen gut steuerbar.

Zum Beispiel kann die Polymerisation in Form einer Dispersion des noch nicht polymerisierten biologischen Materials in einem inerten, flüssigen Medium wie z.B. aliphatischen, cycloaliphatischen und aromatischen Kohlenwasserstoffen, funktionellen Derivaten derselben oder auch Siliconölen, Paraffinölen, durchgeführt werden, wobei in Abhängigkeit von der Verteilung, die durch Rühren, Einleiten von Inertgas, z.B. Stickstoff, oder sonstiges Zudosieren der wäßrigen Phase, z.B. durch Einpumpen oder Einsprühen in die inerte Phase erreicht werden kann, die Größe der Perlen sehr variabel ist. Weiterhin kann durch Zusätze zur Wasser- oder Inertphase deren Viskosität, Dichte, Oberflächenspannung usw. verändert werden, was ebenfalls den Perldurchmesser beeinflußt.

Es sind auf diese Weise mittlere Perldurchmesser von 0,05 mm bis 5 mm möglich, wobei Perlen größeren Durchmesser, z.B. größer 2 mm, der gewünschten Reaktion einen hohen Diffusionswiderstand entgegensetzen. Vorteilhaft sind besonders Perlen eines mittleren Durchmessers von 0,2 mm bis 2 mm.

Neben der Einfachheit der Herstellung der Perlen ergeben sich Vorteile für die Immobilisierung hinsichtlich der Handhabung unter Sterilbedingungen des Biomaterials, da das Verfahren vom Mischen bis zum fertigen immobilisierten Material in einem geschlossenen System durchgeführt werden kann. Das immobilisierte Biomaterial kann anschließend leicht durch Filtration gewonnen werden. Es ist jedoch auch möglich, die gesamte Mischung unter 0°C abzukühlen, um das Material dadurch längere Zeit lagern zu können. Das Abkühlen kann auch über den Erstarrungspunkt des äußeren Mediums hinaus durchgeführt werden, wobei dessen Erstarrungspunkt ebenfalls durch Zusätze oder Beimengen von anderen Stoffen erniedrigt werden kann.

Le A 22 947

Die Erfindung betrifft auch ein Verfahren zur Herstellung von immobilisiertem biologischem Material durch Einschluß in polymerisierte Verbindungen, dadurch gekennzeichnet, daß eine wäßrige Phase, die sowohl das biologische Material als auch höhermolekulare, polymerisierbare Verbindungen enthält, in einer zweiten, nichtwäßrigen Inertphase zu Perlen dispergiert und diese perlförmige Wasserphase polymerisiert wird.

Ein solches Verfahren zur Herstellung von immobilisiertem biologischem Material durch Einschluß in polymerisierte Verbindungen, ist dadurch gekennzeichnet, daß eine wäßrige Lösung oder Dispersion eines biologischen Materials mit einer wäßrigen Lösung von gut wasserlöslichen, höhermolekularen, polymerisierbaren Verbindungen, die zwei oder mehr polymerisierbare funktionelle Gruppen pro Molekül und ein Molekulargewicht von über 400 besitzen, gemischt wird und diese Mischung in einem flüssigen inerten Medium zu Perlen zerteilt und polymerisiert wird.

Als inertes Medium können insbesondere mit Wasser nicht mischbare Stoffe wie aliphatische, cycloaliphatische und aromatische Kohlenwasserstoffe sowie Derivate derselben, Siliconöle, Paraffinöle bzw. auch Gemische derselben untereinander, verwendet werden.

Das Gewichtsverhältnis Wasserphase zu Inertphase sollte zwischen 1:1 und 1:20 liegen.

Le A 22 947

Die Zerteilung der wäßrigen Phase zu Perlen kann durch Rühren und/oder durch Durchleiten von Inertgas oder durch Einsprühen der wäßrigen Phase in die Inertphase erfolgen.

Die Polymerisation durch Bestrahlung mit aktinischem Licht kann unter Zusatz von in der Inertphase schlechtlöslichen oder unlöslichen Photosensibilisatoren erfolgen.

Zur Immobilisierung werden wasserlösliche, höhermolekulare, polymerisierbare Verbindungen, die zwei oder mehr polymerisierbare funktionelle Gruppen pro Molekül und ein Molekulargewicht von über 400, insbesondere zwischen 1.000 und 10.000, besitzen, insbesondere Verbindungen mit ethylenisch ungesättigten Gruppen, verwendet.

Ihrem Aufbau nach lassen sie sich als Verbindungen beschreiben, die aus der Reaktion von höhermolekularen, hydrophilen Verbindungen mit Verbindungen, die polymerisierbare ethylenisch ungesättigte Gruppen enthalten, entstehen. Beide Reaktionspartner müssen dabei funktionelle Gruppen enthalten, die erlauben, sie chemisch mit- oder untereinander zu verknüpfen bzw. mit Hilfe von di- oder mehrfunktionellen Reagentien mit- oder untereinander zu verknüpfen.

Beispielsweise sind solche höhermolekularen, hydrophilen Verbindungen Polyethylenglycole, Ethylenoxid-Propylenoxid-Block- und Mischpolymerisate, alkoxylierte,

Le A 22 947

besonders ethoxylierte zwei- oder mehrwertige Alkohole sowie gut wasserlösliche Polymerisate, z. B. ganz oder teilweise verseifte Polyvinylacetate, gut wasserlösliche Polykondensate wie z. B. Polyester, hergestellt aus obigen höhermolekularen, hydrophilen Verbindungen mit Di- bzw. Polycarbonsäuren und gut wasserlöslichen Polyadditionsverbindungen wie z. B. Polyetherpolyurethane, hergestellt aus obigen höhermolekularen, hydrophilen Verbindungen mit Di- bzw. Polyisocyanaten.

Ferner können die in den obigen genannten höhermolekularen, hydrophilen Verbindungen enthaltenen Hydroxylgruppen ganz oder teilweise nach üblichen Verfahren, wie z. B. Umsetzung mit Ammoniak, in Aminogruppen umgewandelt sein.

Als Verbindungen mit polymerisierbaren, ethylenisch ungesättigten Gruppen sind z. B. geeignet: ungesättigte Carbonsäuren, Säurechloride, Dicarbonsäuren, Säureanhydride oder funktionelle Derivate derselben.

Bevorzugt sind Acrylsäure, Methacrylsäure, Crotonsäure, Acrylsäurechlorid, Methacrylsäurechlorid, Itaconsäure, Fumarsäure, Maleinsäure, Maleinsäureanhydrid, Itaconsäureanhydrid, Glycidylacrylat, Glycidylmethacrylat, Hydroxyethylacrylat, Hydroxypropylacrylat, Hydroxyethylmethacrylat, Hydroxypropylmethacrylat, Isocyanatoethylacrylat, Isocyanatoethylmethacrylat, 4-Isocyanato-3-methyl-but-2-ylacrylat.

Le A 22 947

Die Verknüpfung der höhermolekularen, hydrophilen Verbindungen mit den polymerisierbaren, ethylenisch ungesättigten Verbindungen ist somit unterschiedlichster Natur, beispielsweise ergeben sich Ester-, Ether-, Urethan-, Amid-, Amin- und Harnstoffbindungen. Die Herstellung der Verknüpfung kann dabei nach literaturbekannten Verfahren erfolgen, d. h. beispielsweise durch Umsetzung der Hydroxyl- oder Aminogruppen mit Säuren, Säurehalogeniden oder Säureanhydriden zu den entsprechenden Estern oder Amiden, mit Epoxiden zu den entsprechenden Ethern oder Aminen oder mit Isocyanaten zu den entsprechenden Urethanen oder Harnstoffen.

Ferner kann neben der direkten Verknüpfung der höhermolekularen, hydrophilen Verbindungen mit den Verbindungen, die die ethylenisch ungesättigten Gruppen enthalten, die Verknüpfung auch mittels bi- oder mehrfunktioneller Reagentien erfolgen.

Beispiele solcher bi- oder mehrfunktioneller Reagentien sind di- oder mehrfunktionelle Isocyanate wie Isophorondiisocyanat, Toluylendiisocyanat, Hexamethylendiisocyanat, Biuretgruppenhaltige Polyisocyanate (z. B. Desmodur N, Umsetzungsprodukt von Hexamethylendiisocyanat mit Wasser), Polyisocyanate, die aus der Umsetzung von Diisocyanaten mit mehrwertigen Alkoholen entstehen (z. B. Desmodur L, Umsetzungsprodukt von Toluylendiisocyanat mit Trimethylolpropan) sowie Di- oder Polyepoxide wie z. B. Bisphenol-A-digylcidylether oder Hexahydrophthalsäurediglycidylester. Die Verknüpfung dieser Reagentien erfolgt dabei z. B. mit den Hydroxylgruppen der höher-

Le A 22 947

molekularen, hydrophilen Verbindung und hydroxylgruppenhaltigen Verbindungen, die polymerisierbare, ethylenisch ungesättigte Gruppen enthalten, unter Urethan- bzw. Etherbildung.

Zur Herstellung der erfindungsgemäßen Verbindungen können die verschiedenen Verknüpfungsprinzipien auch untereinander kombiniert werden.

Art und Anteil der hydrophilen, höhermolekularen Verbindungen, der Verbindungen, die die ethylenisch ungesättigten Gruppen tragen sowie ggf. der di- oder mehrfunktionellen Reagentien müssen jedoch so gewählt werden, daß die daraus hergestellten erfindungsgemäßen, höhermolekularen, polymerisierbaren Verbindungen so hydrophil sind, daß die gute Wasserlöslichkeit und große Aufnahmekapazität für wäßrige Lösungen oder Dispersionen an biologischem Material gegeben ist.

Als bevorzugte wasserlösliche, höhermolekulare, polymerisierbare Verbindungen seien genannt:
- Verbindungen, die aus Polyetherpolyolen, deren Hydroxylgruppen teilweise mit ungesättigten Carbonsäuren verestert und zum anderen Teil mit isocyanatgruppenhaltigen Derivaten ungesättigter Carbonsäuren umgesetzt sind, hergestellt sind.

Besonders bevorzugt seien genannt:
- Verbindungen, die aus Polyethylenglykolen mit einem Molekulargewicht größer 400, deren Hydroxylgruppen teilweise mit Acrylsäure oder Methacrylsäure verestert

Le a 22 947

und zum anderen Teil mit Isocyanatoethylmethacrylat
und/oder 4-Isocyanato-3-methyl-but-2-ylacrylat umgesetzt sind, hergestellt sind.

Bei der Herstellung können die Hydroxylgruppen der Polyetherpolyole zunächst teilweise mit den isocyanatgruppenhaltigen Derivaten ungesättigter Carbonsäuren umgesetzt
werden und anschließend zum anderen Teil mit den ungesättigten Carbonsäuren verestert werden, bevorzugt ist
jedoch, zuerst die teilweise Umsetzung mit den ungesättigten Carbonsäuren und anschließend die Umsetzung
des anderen Teils der Hydroxylgruppen mit den isocyanatgruppenhaltigen Derivaten ungesättigter Carbonsäuren.

Weiterhin seien als bevorzugte, wasserlösliche, höhermolekulare, polymerisierbare Verbindungen genannt:
- Verbindungen, die aus Polyetherpolyolen, deren Hydroxylgruppen teilweise mit ungesättigten Carbonsäuren
  verestert und zum anderen Teil mit di- oder mehrfunktionellen Isocyanaten umgesetzt sind, hergestellt sind.

Besonders bevorzugt hierbei seien genannt:
- Verbindungen, die aus Polyethylenglykolen mit einem
  Molekulargewicht größer 400, deren Hydroxylgruppen
  teilweise mit Acrylsäure oder Methacrylsäure verestert
  und zum anderen Teil mit Isophorondiisocyanat, Toluylendiisocyanat, biuretgruppenhaltigen Polyisocyanaten oder Polyisocyanaten, die aus der Umsetzung von
  Diisocyanaten mit mehrwertigen Alkoholen entstehen,
  hergestellt sind.

Le A 22 947

Bei der Herstellung können die Hydroxylgruppen der Polyetherpolyole zunächst teilweise mit den di- oder mehrfunktionellen Isocyanaten umgesetzt werden und anschließend zum anderen Teil mit ungesättigten Carbonsäuren verestert werden. Vorteilhafter ist es jedoch, zuerst die teilweise Veresterung und anschließend die Umsetzung mit den di- oder mehrfunktionellen Isocyanaten durchzuführen.

Die Erfindung betrifft also ein Verfahren, das dadurch gekennzeichnet ist, daß die gut wasserlöslichen, höhermolekularen, polymerisierbaren Verbindungen aus Polyetherpolyolen, deren Hydroxylgruppen teilweise mit ungesättigten Carbonsäuren verestert und zum anderen Teil mit di- oder mehrfunktionellen Isocyanaten umgesetzt sind, hergestellt sind.

Das Verfahren ist dadurch gekennzeichnet, daß

a)   die Polyetherpolyole Polyethylenglykole mit einem MW von 400 und größer sind,

Die Polymerisation kann unter Inertgas durchgeführt und in Gegenwart üblicher Radikalstarter wie Azo-bis(isobutyronitril), t-Butylperoctoat, Benzoylperoxid, Dicyclohexylperoxydicarbonat, Methylethylketonperoxid, Cumolhydroperoxid, Acetyl-cyclohexansulfonyl-peroxid, Dicumylperoxid, Kaliumperoxodisulfat oder Ammoniumperoxodisulfat sowie durch Redoxsysteme wie Kaliumperoxodisulfat-Riboflavin, Kaliumperoxodisulfat-Natriumbisulfit, Wasserstoffperoxid-Verbindungen des zweiwertigen Eisens

Le A 22 947

erfolgen. Als Beschleuniger können ebenfalls zahlreiche Verbindungen dienen, z. B. N,N,N',N'-Tetramethylethylendiamin oder ß-Dimethylaminopropionitril.

Eine weitere Möglichkeit der Polymerisation besteht in der Bestrahlung der Mischung mit aktinischem Licht. Es eignen sich beispielsweise Hochdruckquecksilberlampen, Niederdruckquecksilberlampen, Fluoreszenzlampen, Xenonlampen, Kohlelichtbögen sowie Sonnenbestrahlung. Eine Bestrahlung mit Elektronenstrahlen oder Gammastrahlen ist ebenfalls möglich, jedoch muß mit einer gewissen Schädigung des biologischen Materials gerechnet werden. Ferner kann die Photopolymerisation durch Photosensibilisatoren beschleunigt werden. Es können bekannte Photosensibilisatoren wie $\alpha$-Carbonylalkohole, z.B. Benzoin oder Acetoin, Acyloinether wie Benzoinmethylether, Benzoinethylether, Benzoinisopropylether, $\alpha$-substituierte Acyloine wie $\alpha$-Methylbenzoin und $\alpha$-Methoxybenzoin u.a. sowie durch ionische Gruppen wie z.B. Carbonsäure-, Sulfonsäure- oder Aminogruppen modifizierte und dadurch wasserlöslich gemachte Derivate verwendet werden.

Des weiteren können polycyclische aromatische Verbindungen wie Naphthol und Hydroxyanthracen, Azoamide wie beispielsweise 2-Cyano-2-butylazoformamid sowie Metallsalze wie Uranylnitrat und Eisenchlorid, wie auch Mercaptane, Disulfide, Halogenide und Farbstoffe verwendet werden.

Durch die hohe Aufnahmekapazität für Wasser - besonders gut eignen sich Gewichtsverhältnisse von wäßriger Lösung

Le A 22 947

Bei der Herstellung können die Hydroxylgruppen der Polyetherpolyole zunächst teilweise mit den di- oder mehrfunktionellen Isocyanaten umgesetzt werden und anschließend zum anderen Teil mit ungesättigten Carbonsäuren verestert werden. Vorteilhafter ist es jedoch, zuerst die teilweise Veresterung und anschließend die Umsetzung mit den di- oder mehrfunktionellen Isocyanaten durchzuführen.

Die Erfindung betrifft also ein Verfahren, das dadurch gekennzeichnet ist, daß die gut wasserlöslichen, höhermolekularen, polymerisierbaren Verbindungen aus Polyetherpolyolen, deren Hydroxylgruppen teilweise mit ungesättigten Carbonsäuren verestert und zum anderen Teil mit di- oder mehrfunktionellen Isocyanaten umgesetzt sind, hergestellt sind.

Das Verfahren ist dadurch gekennzeichnet, daß

a) die Polyetherpolyole Polyethylenglykole mit einem MW von 400 und größer sind,

Die Polymerisation kann unter Inertgas durchgeführt und in Gegenwart üblicher Radikalstarter wie Azo-bis(isobutyronitril), t-Butylperoctoat, Benzoylperoxid, Dicyclohexylperoxydicarbonat, Methylethylketonperoxid, Cumolhydroperoxid, Acetyl-cyclohexansulfonyl-peroxid, Dicumylperoxid, Kaliumperoxodisulfat oder Ammoniumperoxodisulfat sowie durch Redoxsysteme wie Kaliumperoxodisulfat-Riboflavin, Kaliumperoxodisulfat-Natriumbisulfit, Wasserstoffperoxid-Verbindungen des zweiwertigen Eisens

<u>Le A 22 947</u>

erfolgen. Als Beschleuniger können ebenfalls zahlreiche Verbindungen dienen, z. B. N,N,N',N'-Tetramethylethylendiamin oder ß-Dimethylaminopropionitril.

Eine weitere Möglichkeit der Polymerisation besteht in der Bestrahlung der Mischung mit aktinischem Licht. Es eignen sich beispielsweise Hochdruckquecksilberlampen, Niederdruckquecksilberlampen, Fluoreszenzlampen, Xenonlampen, Kohlelichtbögen sowie Sonnenbestrahlung. Eine Bestrahlung mit Elektronenstrahlen oder Gammastrahlen ist ebenfalls möglich, jedoch muß mit einer gewissen Schädigung des biologischen Materials gerechnet werden. Ferner kann die Photopolymerisation durch Photosensibilisatoren beschleunigt werden. Es können bekannte Photosensibilisatoren wie $\alpha$-Carbonylalkohole, z.B. Benzoin oder Acetoin, Acyloinether wie Benzoinmethylether, Benzoinethylether, Benzoinisopropylether, $\alpha$-substituierte Acyloine wie $\alpha$-Methylbenzoin und $\alpha$-Methoxybenzoin u.a. sowie durch ionische Gruppen wie z.B. Carbonsäure-, Sulfonsäure- oder Aminogruppen modifizierte und dadurch wasserlöslich gemachte Derivate verwendet werden.

Des weiteren können polycyclische aromatische Verbindungen wie Naphthol und Hydroxyanthracen, Azoamide wie beispielsweise 2-Cyano-2-butylazoformamid sowie Metallsalze wie Uranylnitrat und Eisenchlorid, wie auch Mercaptane, Disulfide, Halogenide und Farbstoffe verwendet werden.

Durch die hohe Aufnahmekapazität für Wasser - besonders gut eignen sich Gewichtsverhältnisse von wäßriger Lösung

Le A 22 947

oder Suspension des biologischen Materials zum Polymer zwischen 4:1 und 20:1 - die das immobilisierte Material vor und nach der Polymerisation besitzt, ergeben sich auch Vorteile hinsichtlich der Wirtschaftlichkeit des Verfahrens. Dadurch können einerseits große Mengen an wäßrigen Lösungen oder Dispersionen des biologischen Materials in einer relativ kleine Menge der polymerisierbaren Verbindungen eingeschlossen werden, andererseits können dadurch Fermenterbrühen direkt oder nach Konzentrierung z.B. durch Mikrofiltration oder Zentrifugieren immobilisiert werden.

Desweiteren ist es jedoch vor und nach der Immobilisierung auch möglich, geringere Menge an Wasser oder wäßrigen Lösungen zu verwenden, sowie einen Teil des Anteils an Wasser gegen andere Flüssigkeiten wie z.B. Alkohole zu ersetzen.

Es ist auch möglich, die immobilisierten biologischen Materialien in anderen als wäßrigen Lösungen, z.B. in aliphatischen oder aromatischen Kohlenwasserstoffen, einzusetzen.

Die beschriebenen Verfahren sind dadurch gekennzeichnet, daß Zellen oder Enzyme in Gegenwart von Desinfektionsmitteln, Bakteriziden oder Fungiziden immobilisiert werden, somit chemisch sterilisiert in den Bioreaktor überführt werden und die Sterilisationsmittel vor Anlauf der Reaktion aus dem geschlossenen, sterilen Reaktorsystem ausgewaschen werden.

Le A 22 947

Die nach vorstehendem Verfahren immobilisierten biologischen Materialien, insbesondere Zellen oder Enzyme, lassen sich als Biokatalysatoren zu Biotransformationen einsetzen.

Interessante Mikroorganismen für die erfindungsgemäße Immobilisierung sind die folgenden:

Aspergillus niger, Gluconobacter suboxydans, Gluconobacter oxydans, Escherichia coli, Saccharomyces cerevisiae, Protaminobacter rubrum, Serratia plymuthica, Pseudomonas putida, Cunninghamella elegans, Clostridien wie z.B. Clostridium thermoaceticum, Clostridium kluyveri, Clostridium butyricum, Clostridium sporogenes, Bacillus licheniformis, Streptomyces olivaceus.

Le A 22 947

## Beispiele

## Beispiel 1

Immobilisierung von Alkoholdehydrogenase

48,7 g Polyethylenglykol mit einem mittleren Molekulargewicht von 800 (hergestellt von den Chemischen Werken Hüls) wurde mit

4,7 g Acrylsäure unter Zusatz von

0,54 g p-Toluolsulfonsäure und

50 mg Di-tert.-butylhydrochinon sowie

50 mg p-Methoxyphenol in Toluol verestert. 25,0 g dieses Esters wurden mit

3,9 g Isocyanatoethylmethacrylat (hergestellt von Dow Chemicals) unter Zusatz von

4 mg Desmorapid SO (Rheinchemie Rheinau GmbH) umgesetzt.

1 g des so erhaltenen polymerisierbaren Harzes wurden mit 50 mg Irgacure 651 (Ciba-Geigy) gemischt und mit 2 g Phosphat-Puffer (pH 7, 0,01 M) versetzt. In dieser Lösung wurde 200 mg Alkoholdehydrogenase (aus Hefe, Fa. Boehringer Mannheim, enthaltend 120 mg Enzymprotein der spezifischen Aktivität 400 U/mg) gelöst, aus der Lösung ein 500 µm dicker Film gezogen und mittels einer Quecksilberhochdrucklampe 10 min. bestrahlt, so daß ein fester Film entstand.

Ein Viertel des so erhaltenen Präparates (entsprechend 30 mg Alkohol-Dehydrogenase) wurde in kleine Stücke geschnitten und in einem 100 ml Erlenmeyerkolben bis

Le A 22 947

zu einem Endvolumen von 20 ml mit 0,1 M Tris. HCl,
pH 8,6 enthaltend 142 mg NAD, Li-Salz (Fa. Boehringer,
Mannheim), 0,1 M Semicarbazid, 5 % Ethanol p.A. versetzt.

Der Reaktionslösung wurden zu bestimmten Zeiten Proben
entnommen und nach Verdünnung das UV-VIS Spectrum des
entstehenden NADH gemessen. Nach etwa 50 min. war die
Gesamtmenge an NAD zu NADH reduziert worden mit einer
Initialgeschwindigkeit von 8,1 µ Mol/min. Die immobilisierte Alkohol-Dehydrogenase besitzt somit eine spezifische Aktivität von 0,28 U/mg immobilisiertes Enzymprotein. Das immobilisierte Präparat konnte wiederholt verwendet werden.

Beispiel 2

Immobilisierung von Alkoholdehydrogenase

1 g des in Beispiel 1 erhaltenen polymerisierbaren Harzes wurden mit 50 mg 1,2-Diphenyl-2-hydroxy-3$\sqrt{N}$-(N-
methyl)pyrrolidinium7-propan-1-on-methylsulfat gemischt
und mit 15 g Phosphat-Puffer (0,01 M, pH 7) versetzt.
In dieser Lösung wurde 200 mg Alkoholdehydrogenase (aus
Hefe, Fa. Boehringer, Mannheim, enthaltend 120 mg Enzymprotein, 400 U/mg) gelöst und die Lösung in 100 g
einer Mischung aus Silikonöl/Paraffinöl mit einer
spez. Dichte von 0,9 g/cm³ getropft, wobei sich durch
intensives Rühren und Durchleiten von Stickstoff Perlen
ausbildeten, die durch 15 minütige Bestrahlung mittels
einer Hg-Hochdrucklampe polymerisiert wurden.

Le A 22 947

1,0 g der so erhaltenen Perlen wurden in 50 ml Erlenmeyerkolben mit 10 ml 0,1 M Kaliumphosphat pH 8,5, 10 mM NADH, 1 % Acetaldehyd auf der Rundschüttelmaschine, 230 rpm, bei 30°C inkubiert.

Nach 6 h waren etwa 50 % des eingesetzten NADH, 50 µMol, zu NAD oxidiert, die Initialgeschwindigkeit betrug dabei 0,23 µmol/min.

Beispiel 3

Immobilisierung von Lipase

1 g des polymerisierbaren Harzes aus Beispiel 1 wurde mit 50 mg Irgacure 651 (Ciba-Geigy) gemischt und mit 3 g Phosphatpuffer (0,01 M, pH 7) versetzt. In dieser Lösung wurde 0,8 g Lipase (aus Candida cylindracea, Sigma) dispergiert. Die gesamte Mischung wurde anschließend auf ein Polyamidgewebe (Monodur PA 250 N, Verseidag-Industrietextilien GmbH) verstrichen und mittels einer Quecksilberhochdrucklampe 10 min. bestrahlt, so daß ein fester Film, verstärkt durch das Polyamidgewebe, entstand.

160 mg Lipase eingeschlossen in obiges Polymer, entsprechend 72 cm² zerschnittener Folie, wurden in 40 ml $H_2O$ mit 2 g Sucrose-Palmitat-Stearat 15 (Fa. Serva, Heidelberg) bei 25°C inkubiert. Freiwerdende Fettsäure wurde mit 0,1 N NaOH per pH-Stat (pH 7,1) titriert.

Le A 22 947

Die Aktivität immobilisierte Lipase wurde zu 5,8 mU/mg trockenes Lipase-Präparat errechnet (1 U = 1 µMol freigesetzte Fettsäure pro Minute).

Beispiel 4

Immobilisierung von Lipase

197 g Oktaethylenglykol wurden mit 36 g Acrylsäure unter Zusatz von 2 g p-Toluolsulfonsäure und 0,35 g di-tert.-butylhydrochinon sowie 0,35 g p-Methoxyphenol in Toluol verestert. 100 g des so erhaltenen Esters wurden mit 47 g Isophorondiisocyanat und 90 mg Desmorapid SO umgesetzt.

5 g des so erhaltenen polymerisierbaren Harzes wurden in 2 g 0,01 M Kaliumphosphat-Puffer, pH 7,0, gelöst, mit 6 g einer Lipase-Suspension (Lipase aus Candida cylindracea, Fa. Sigma; 17 % Feststoffgehalt) gemischt und mit 100 mg Irgacure 651 (Ciba-Geigy) versetzt. Diese wäßrige Lipase-Mischung wurde auf ein Polyamid-Stützgewebe (Monodur PA 250 N, Verseidag-Industrietextilien GmbH) verstrichen und durch 10-minütige Bestrahlung mit einer Hg-Hochdrucklampe polymerisiert.

72 cm² der so erhaltenen immobilisierten Lipase werden in 40 ml Wasser pH 7,1 eingebracht und mit 1,0 g Sucrose-Palmitat-Stearat 15 (Fa. Serva, Heidelberg) bei 25°C versetzt. Freiwerdende Fettsäuren werden am pH-Staten mit 0,1 N NaOH titriert. Pro Stunde werden

Le A 22 947

2,74 ml 0,1 N NaOH verbracht, was 274 µMol freier
Fettsäure entspricht. Pro cm² immobilisierter Lipase-
Folie errechnen sich 0,065 µMol/min freiwerdende
Fettsäure.

Beispiel 5

Immobilisierung von Esterase

25,0 g des hergestellten Esters aus Beispiel 1 wurden
mit 2,3 g Desmodur N (Bayer AG, Leverkusen) und
1,4 g Isophorondiisocyanat unter Zusatz von 7,5 mg Desmorapid SO (Rheinchemie Rheinau GmbH) umgesetzt.

5 g des so erhaltenen polymerisierbaren Harzes,
250 mg Irgacure 651 (Ciba-Geigy), 8,5 g Phosphatpuffer
(0,01 M, pH 7) und 3 ml Esterase-Suspension (aus
Schweineleber, Boehringer Mannheim GmbH) wurden gemischt und auf einem Polyamidgewebe (Monodur PA 250 N,
Verseidag-Industrietextilien GmbH) verstrichen.
Nach Belichten mittels einer Quecksilberhochdrucklampe
wurde ein durch das Polyamidgewebe verstärkter Film
erhalten.

36 cm² der so erhaltenen Esterase-Folie wurden in
45 ml 0,1 M Natriumphosphat pH 7,0 mit 5 ml einer
100 mM Lösung von Nitroterephthalsäuredimethylester
in Methanol bei 30°C auf einer Rotationsschüttelmaschine inkubiert.

Le A 22 947

Die Analyse der Reaktionslösung erfolgte per HPLC. Nach 48 h ist keine Ausgangsverbindung mehr nachweisbar. Es bildeten sich die beiden Nitroterephthalsäuremonoester sowie in geringer Menge auch Nitroterephthalsäure.

Beispiel 6

Immobilisierung von L-Lactat-Dehydrogenase

5 g des in Beispiel 5 verwendeten polymerisierbaren Harzes wurden mit 250 mg Phenylglyoxylsäure, 5 g Phosphatpuffer (0,01 M, pH 7) und 3 ml L-Lactat-Dehydrogenase-Suspension (aus Schweinemuskel, Boehringer Mannheim GmbH, enthaltend 10 mg/ml Enzymprotein, spezifische Aktivität ca. 550 U/mg) gemischt und analog Beispiel 5 zu einem Film verarbeitet.

Die Hälfte des so erhaltenen Films, enthaltend etwa 15 mg Enzymprotein, wurde zerschnitten und in einem 100 ml Erlenmeyerkolben mit 20 ml 0,1 M Kaliumphosphat-Puffer (pH 8,0), 10 mM NADH, 0,05 M Pyruvat-Na-Salz inkubiert. Die Temperatur betrug 30°C, die Reaktionslösung wurde auf einer Schüttelmaschine mit 200 Bewegungen pro Minute geschüttelt.

Von Zeit zu Zeit wurden Proben entnommen und nach Verdünnung photometrisch die NADH-Abnahme gegen eine Blindprobe ohne immobilisierte L-Lactat-Dehydrogenase

Le A 22 947

bestimmt. Nach 60 Minuten war die gesamte Menge an NADH zu NAD oxidiert worden. Die Initialgeschwindigkeit wurde zu 3,9 µMol/min bestimmt, was einer spezifischen Aktivität von 0,26 U/mg eingeschlossenes Enzymprotein entspricht.

Beispiel 7

Immobilisierung von L-Lactat Dehydrogenase

1 g des in Beispiel 5 erhaltenen polymerisierbaren Harzes wurden mit 50 mg 1,2-Diphenyl-2-hydroxy-3/N̄-(N-methyl)pyrrolidinium/-propan-1-on-methylsulfat gemischt und mit 1,5 g Phosphat-Puffer (0,01 M, pH 7) versetzt. In dieser Lösung wurde 0,6 ml L-Lactat-Dehydrogenase (aus Schweinemuskel, Fa. Boehringer, Mannheim, enthaltend 10 mg/ml Enzymprotein, ca. 550 U/mg) gelöst und die Lösung in 100 g einer Mischung aus Silikonöl/Paraffinöl mit einer spez. Dichte von 0,9 g/cm³ getropft, wobei sich durch intensives Rühren und Durchleiten von Stickstoff Perlen ausbildeten, die durch 15 minütige Bestrahlung mittels einer Hg-Hochdrucklampe polymerisiert wurden.

Die erhaltenen Perlen wurden in einem 100 ml Erlenmeyerkolben mit 20 ml 0,1 M Kaliumphosphat-Puffer, (pH 8,0), 10 mM NADH 0,05 M Pyruvat-Na-Salz bei 30°C, 200 rpm, inkubiert.

Entnommene Proben wurden nach Verdünnung photometrisch auf die NADH-Abnahme gegen eine Blindprobe getestet.

Le A 22 947

Nach 3 h war die gesamte Menge an NADH zu NAD oxidiert.
Die Initialgeschwindigkeit betrug 1,4 μMol/min.

Beispiel 8

Immobilisierung von Bäckerhefe

100,0 g Polyethylenglykol mit einem mittleren Molekulargewicht von 1.000 (Chemische Werke Hüls) wurde
mit 7,2 g Acrylsäure unter Zusatz von 0,92 g
p-Toluolsulfonsäure und 0,1 g Di-tert.-butylhydro-
chinon sowie 0,1 g p-Methoxyphenol in Toluol verestert.

75,0 g des so erhaltenen Esters wurde mit 8,3 g Isophorondiisocyanat unter Zusatz von 9 mg Desmorapid S0
umgesetzt.

5,0 g des polymerisierbaren Harzes wurde mit 0,1 g
Irgacure 651 (Ciba-Geigy) und 1,0 g Pufferlösung
(0,01 M, pH 7) gemischt. Zu dieser Lösung wurde eine
Mischung aus 13,7 g Bäckerhefe (25 % Feststoffgehalt)
und 13,7 g Pufferlösung (0,01 M, pH 7) gegeben. Die
so erhaltene Mischung wurde nach Ausstreichen auf ein
Polyamidgewebe mittels einer Quecksilberhochdrucklampe
belichtet, so daß ein fester Film entstand.

36 cm² des so erhaltenen Films wurden auf ihre NADH-
Oxidase Aktivität getestet. Dazu wurde der Film zerschnitten und in einem 200 ml Erlenmeyerkolben bis zu bestimmt. Nach 60 Minuten war die gesamte Menge an NADH zu NAD oxidiert worden. Die Initialgeschwindigkeit wurde zu 3,9 μMol/min bestimmt, was einer spezifischen Aktivität von 0,26 U/mg eingeschlossenes Enzymprotein entspricht.

Beispiel 7

Immobilisierung von L-Lactat Dehydrogenase

1 g des in Beispiel 5 erhaltenen polymerisierbaren Harzes wurden mit 50 mg 1,2-Diphenyl-2-hydroxy-3/N̄-(N-methyl)pyrrolidinium7-propan-1-on-methylsulfat gemischt und mit 1,5 g Phosphat-Puffer (0,01 M, pH 7) versetzt. In dieser Lösung wurde 0,6 ml L-Lactat-Dehydrogenase (aus Schweinemuskel, Fa. Boehringer, Mannheim, enthaltend 10 mg/ml Enzymprotein, ca. 550 U/mg) gelöst und die Lösung in 100 g einer Mischung aus Silikonöl/Paraffinöl mit einer spez. Dichte von 0,9 g/cm³ getropft, wobei sich durch intensives Rühren und Durchleiten von Stickstoff Perlen ausbildeten, die durch 15 minütige Bestrahlung mittels einer Hg-Hochdrucklampe polymerisiert wurden.

Die erhaltenen Perlen wurden in einem 100 ml Erlenmeyerkolben mit 20 ml 0,1 M Kaliumphosphat-Puffer, (pH 8,0), 10 mM NADH  0,05 M Pyruvat-Na-Salz bei 30°C, 200 rpm, inkubiert.

Entnommene Proben wurden nach Verdünnung photometrisch auf die NADH-Abnahme gegen eine Blindprobe getestet.

Le A 22 947

Nach 3 h war die gesamte Menge an NADH zu NAD oxidiert. Die Initialgeschwindigkeit betrug 1,4 µMol/min.

Beispiel 8

Immobilisierung von Bäckerhefe

100,0 g Polyethylenglykol mit einem mittleren Molekulargewicht von 1.000 (Chemische Werke Hüls) wurde mit 7,2 g Acrylsäure unter Zusatz von 0,92 g p-Toluolsulfonsäure und 0,1 g Di-tert.-butylhydrochinon sowie 0,1 g p-Methoxyphenol in Toluol verestert.

75,0 g des so erhaltenen Esters wurde mit 8,3 g Isophorondiisocyanat unter Zusatz von 9 mg Desmorapid S0 umgesetzt.

5,0 g des polymerisierbaren Harzes wurde mit 0,1 g Irgacure 651 (Ciba-Geigy) und 1,0 g Pufferlösung (0,01 M, pH 7) gemischt. Zu dieser Lösung wurde eine Mischung aus 13,7 g Bäckerhefe (25 % Feststoffgehalt) und 13,7 g Pufferlösung (0,01 M, pH 7) gegeben. Die so erhaltene Mischung wurde nach Ausstreichen auf ein Polyamidgewebe mittels einer Quecksilberhochdrucklampe belichtet, so daß ein fester Film entstand.

36 cm² des so erhaltenen Films wurden auf ihre NADH-Oxidase Aktivität getestet. Dazu wurde der Film zerschnitten und in einem 200 ml Erlenmeyerkolben bis zu

Le A 22 947

einem Endvolumen von 50 ml mit 0,05 M Kaliumphosphat-Puffer, pH 8,0, sowie mit 360 mg NADH (Grad I, Boehringer Mannheim) versetzt und auf einer Rotationsschüttelmaschine mit 200 rpm gegen eine Blindprobe ohne immobilisierte Bäckerhefe bei 30°C inkubiert.

Die immobilisierten Hefezellen oxidierten NADH mit einer Geschwindigkeit von 5,3 µMol/h, gemessen als Mittelwert über 48 h. Diese Reaktion ist zur NAD-Regenerierung mittels $O_2$ in gekoppelten Enzymsystemen verwendbar.

Beispiel 9

Immobilisierung von Bäckerhefe

218,9 g Polyethylenglykol mit einem mittleren Molekulargewicht von 1.550 (Chemische Werke Hüls) wurde mit 9,8 g Acrylsäure unter Zusatz von 2,3 g p-Toluolsulfonsäure, 0,23 g Di-tert.-butylhydrochinon sowie 0,23 g p-Methoxyphenol in Toluol verestert.

165,8 g des so erhaltenen Esters wurden mit 18,2 g Isocyanatoethylmethacrylat (Dow Chemicals) unter Zusatz von 36 mg Desmorapid SO umgesetzt.

9,0 g dieses polymerisierbaren Harzes wurden mit 90 mg Irgacure 651 gemischt und mit 2,7 g Phosphat-puffer (0,01 M, pH 7) versetzt. Diese so erhaltene Lösung wurde zu einer Mischung aus 20,0 g Bäckerhefe

Le A 22 947

und 20,0 g Phosphatpuffer (0,01 M, pH 7) gegeben, daraus ein 500 µm dicker Film gezogen und durch Bestrahlung mittels einer Quecksilberhochdrucklampe polymerisiert.

36 cm² der so erhaltenen Folie wurden auf NADH-Oxidase Aktivität getestet. Die zerkleinerte Folie wurde in 200 ml Erlenmeyerkolben bis zu einem Endvolumen von 50 ml mit 0,05 M Kaliumphosphat-Puffer, pH 8,0, sowie mit 360 mg NADH (Grad I, Fa. Boehringer, Mannheim) versetzt und auf einer Rotationsschüttelmaschine mit 200 rpm gegen eine Blindprobe der gleichen Zusammensetzung jedoch ohne immobilisierte Bäckerhefe bei 30°C inkubiert. NADH wurde mit einer Geschwindigkeit von 7,4 µMol/h, gemessen als Mittelwert über 48 h, oxidiert.

## Beispiel 10

Immobilisierung von Bäckerhefe

5,0 g des polymerisierbaren Harzes aus Beispiel 8 wurde mit 0,1 g Phenylglyoxylsäure und 1,0 g Pufferlösung (0,01 M, pH 7) gemischt. Zu dieser Lösung wurde eine Mischung aus 13,7 g Bäckerhefe (25 % Feststoffgehalt) und 13,7 g Pufferlösung pH 7 gegeben. Die so erhaltene Mischung wurde anschließend in 200 ml einer Mischung aus Paraffinöl und Siliconöl mit einer spez. Dichte von 0,9 g/cm³ unter Rühren und Durchleiten von Stickstoff in Perlen zerteilt und mittels einer Quecksilberhochdrucklampe polymerisiert.

Le A 22 947

15 g dieser Perlen wurden in einem 200 ml Erlenmeyerkolben mit 50 ml 0,01 M Kaliumphosphat, pH 8,0,
1 mM NADH (Grad II, Fa. Boehringer, Mannheim) bei 25°C
auf einer Rundschüttelmaschine bei 230 rpm inkubiert.

Die NADH-Oxidase Aktivität der immobilisierten Hefezellen wurde über die Abnahme des NADH-Gehalts photometrisch gegen eine Blindprobe ohne Hefezellen bestimmt.

15 g immobilisiertes Präparat besaß eine Aktivität
von 0,24 U (1 U = 1 µMol NADH/min. oxidiert).
Diese Reaktion ist zur NAD-Regenerierung in gekoppelten Enzymsystemen anwendbar.

Beispiel 11

Immobilisierung von Bäckerhefe

9,0 g des polymerisierbaren Harzes aus Beispiel 9
wurden mit 90 mg Phenylglyoxylsäure gemischt und
mit 2,70 g Phosphatpuffer (0,01 M, pH 7) versetzt.

Diese so erhaltene Lösung wurde zu einer Mischung aus
20,0 g Bäckerhefe und 20,0 g Phosphatpuffer (0,01 M,
pH 7) gegeben und daraus analog Beispiel 10 Perlen
hergestellt.

15 g feuchte Perlen wurden in einem 200 ml Erlenmeyerkolben mit 50 ml 0,01 M Kaliumphosphat pH 8,0,
1 mM NADH (Fa. Boehringer, Mannheim) bei 25°C auf

Le A 22 947

einer Rundschüttelmaschine, 230 rpm, inkubiert.
Aufgrund der photometrischen NADH-Bestimmung besaßen 15 g Perlen eine Aktivität von 0,14 U.

Beispiel 12

Immobilisierung von Bäckerhefe

10 g des polymerisierbaren Harzes aus Beispiel 13
wurden mit 4.0 g Phosphatpuffer (0.01 M, pH 7.0) versetzt, sodaß eine Lösung entstand. Diese so erhaltene Lösung wurde zu einer Mischung aus 17.5 g
Bäckerhefe und 17.5 g Phosphatpuffer (0.01 M,
pH 7.0) gegeben. Dann wurde darin 0.2 g Ammoniumpersulfat aufgelöst und mit 0.2 g N,N,N',N'-Tetramethylethylendiamin versetzt. Nach intensivem
Durchmischen polymerisierte die Lösung nach wenigen
Minuten im Becherglas.

Der entstandene Block wurde in kleine Stücke (ungefähr 5 x 5 x 3 mm) zerschnitten und auf NADH-Oxidase
Aktivität getestet, analog Beispiel 9. Dabei wurden
10 g des feuchten Polymerisats in den Test eingesetzt.
NADH wurde mit einer Rate von 19.3 µMol/h oxidiert.

Als Kontrollversuch lief ein Parallelansatz gleicher
Zusammensetzung ohne immobilisierte Bäckerhefe, bei
dem jedoch keine Abnahme des NADH-Gehalts festgestellt
wurde.

Le A 22 947

Beispiel 13

Immobilisierung von Protaminobacter rubrum

110,0 g des Esters aus Beispiel 9 wurden mit
7,4 g Isophorondiisocyanat unter Zusatz von 15 mg
Desmorapid SO umgesetzt.

5,0 g dieses so erhaltenen polymerisierbaren Acrylatharzes wurden mit 50 mg Irgacure 651 gemischt und
mit 2,0 g Phosphatpuffer (0,01 M, pH 7) versetzt,
so daß eine Lösung entstand.

Zur Produktion von Sucrose Mutase wird der Stamm
Protaminobacter rubrum (CBS 574.77) verwendet. Die
Nährlösung besteht aus 5 % Dicksaft, 2 % Maisquellwasser und 0,05 % $(NH_4)_2HPO_4$, der pH-Wert
beträgt 7,2. Mit 1 ml einer Protaminobacter rubrum
Abschwemmung werden in einem 1 l Erlenmeyerkolben
200 ml Nährlösung beimpft. Die Fermentation läuft
15 h bei 31°C auf der Rundschüttelmaschine.

Mit dieser Vorkultur werden in einem 30 Liter Fermenter 20 Liter obiger Nährlösung angeimpft und
16 h bei 31°C fermentiert. Die Fermentationslösung
wurde durch Mikrofiltration um den Faktor 10
konzentriert.

17,5 g dieser konzentrierten Fermenterlösung wurde
mit der obigen Acrylatharzlösung vermischt und auf
ein Polyamidgewebe (Monodur PA 250 N, Verseidag-

Le A 22 947

Industrietextilien GmbH) verstrichen. Nach Bestrahlung für 15 Minuten mittels einer Quecksilberhochdrucklampe entstand ein polyamidverstärkter Film.

Zum Test der immobilisierten Protaminobacter rubrum
Zellen wurde deren Sucrose Mutase Aktivität gemessen.
Hierzu wurden 36 cm² Folie in 50 ml 50 % Sucrose-Lösung
pH 7,0, bei 30°C auf einer Rotationsschüttelmaschine
inkubiert und zu verschiedenen Zeiten Proben genommen.
Die Bestimmung des Umsatzes von Sucrose zu Isomaltulose
erfolgte per HPLC. Die Umsatzrate betrug 0,60 g/h
Sucrose, was 620 U/g Trockenzellen entspricht. 1 µMol/
min umgesetzte Sucrose entspricht 1 U.

**Beispiel 14**

Immobilisierung von Protaminobacter rubrum

Die Anzucht von Protaminobacter rubrum (CBS 574.77)
erfolgte analog Beispiel 13.

20 g des polymerisierbaren Harzes aus Beispiel 9
wurden mit 6 g Phosphatpuffer (0.01 M, pH 7.0) versetzt. Diese so erhaltene Lösung wurde mit 180 g
durch Mikrofiltration etwa um den Faktor 10 konzentrierten Protaminobacter Zellsuspension vermischt.
Nach Auflösen von 0,4 g Ammoniumperoxodisulfat in
dieser Lösung wurden 0,4 g N,N,N',N'-Tetramethylethylendiamin zugesetzt, gut durchgemischt und

Le A 22 947

schnell in mehrere Schalen gegossen, sodaß die Schichtdicke des ausgegossenen Materials etwa 3 mm betrug. Nach wenigen Minuten setzte die Polymerisation ein, die polymerisierten Scheiben wurden in kleine Stücke der Größe 5 x 5 x 3 mm zerschnitten und in eine 500 ml Säule gefüllt.

Nach Einstellen eines Durchflusses von etwa 90 ml/h wurden bei 30°C 72 % der Sucrose einer 50 % Sucroselösung zu Isomaltulose und Nebenprodukten umgesetzt. Eine Erhöhung des Durchflusses auf 135 ml/h führte zu einer Abnahme des Sucroseumsatzes auf 52.7 %.

Beispiel 15

Immobilisierung von Protaminobacter rubrum

Zur Produktion von Sucrose Mutase wird der Stamm Protaminobacter rubrum (CBS 574.77) verwendet, die Anzucht erfolgte analog Beispiel 13.

10 g des in Beispiel 9 erhaltenen polymerisierbaren Acrylatharzes wurden mit 3,0 g Phosphatpuffer (0,01 M, pH 7) und 500 mg Irgacure (Ciba-Geigy) gemischt. Zu dieser Lösung wurden 70 g einer durch Mikrofiltration konzentrierten Fermenterbrühe, die Protaminobacter rubrum Zellen enthält, gegeben und diese Mischung in 460 g eines Siliconöles mit einer spez. Dichte von 0,95 g/cm³ getropft. Durch intensives Rühren und durch Stickstoffeinleitung wurde eine

Le A 22 947

Zerteilung der obigen zellenthaltenden Mischung in
Perlen erreicht, die mittels 2 Quecksilberhochdrucklampen und 1/2-stündiger Bestrahlung polymerisiert wurden.

6,0 g der so erhaltenen Perlen wurden in einem 200 ml
Erlenmyerkolben mit 50 ml 50 % Sucrose-Lösung, pH 7,0,
auf der Rundschüttelmaschine bei 230 rpm inkubiert.
Die Analyse der Reaktionslösung erfolgte per HPLC.
Die immobilisierten Protaminobacter Zellen setzten
0,32 g/h Sucrose um.

Beispiel 16

Immobilisierung von Protaminobacter rubrum

Die Anzucht von Protaminobacter rubrum (CBS 574.77)
erfolgte analog Beispiel 13.

110 g des Esters aus Beispiel 9 wurden mit 7,4 g
Isophorondiisocyanat unter Zusatz von 15 mg Desmorapid SO umgesetzt.

10 g des so erhaltenen polymerisierbaren Acrylatharzes wurden mit 4 g Phosphatpuffer 0,01 M, pH 7,0 und
500 mg 1,2-Diphenyl-2-hydroxy-3/N̄-(N-methyl)pyrrolidi-
nium7-propan-1-on-methylsulfat und 35 g konzentrierter Fermenterbrühe gemischt und zu 300 g eines
Siliconöl-Paraffinöl-Gemisches mit einer spez. Dichte
von 0,9 g/cm³ gegeben und analog Beispiel 15 Perlen
hergestellt.

Le A 22 947

6,0 g der so erhaltenen Perlen wurden analog Beispiel 15 mit 50 % Sucroselösung getestet. Die immobilisierten Protaminobacter Zellen setzten 0,69 g/h
Sucrose um.

Beispiel 17

Immobilisierung von Protaminobacter rubrum

Die Anzuchtvon Protaminobacter rubrum (CBS 574.77) erfolgte analog Beispiel 13.

10 g des polymerisierbaren Harzes aus Beispiel 16 wurden
mit 4 g Phosphatpuffer, (0.01 M, pH 7.0), und 35 g
konzentrierter Fermenterbrühe gemischt und darin 0,3 g
Ammoniumperoxodisulfat gelöst. Diese Mischung wurde
in 2000 g eines Siliconöls der Dichte 0,97 g/cm³, in
dem 0.3 g N,N,N',N'-Tetramethylethylendiamin gelöst
waren, unter starkem Rühren eingetropft, so daß die
wäßrige Mischung zu Perlen zerteilt wurde und innerhalb kurzer Zeit polymerisierte.

Die gesamte Perlmasse wurde in eine 25 cm lange Säule
des Durchmessers 2 cm gepackt, das anhaftende Siliconöl
durch 50 % Sucroselösung verdrängt und die Säule bei
45°C kontinuierlich betrieben.

Bei einem Durchfluß von 28 ml/h wurden 54.5 % der 50 %
Sucroselösung zu Isomaltulose und Nebenprodukten umgesetzt.

Le A 22 947

Patentansprüche

1. Verfahren zur Immobilisierung von biologischem Material durch Einschluß in polymerisierte Verbindungen, dadurch gekennzeichnet, daß wäßrige Lösungen oder Dispersionen des biologischen Materials mit einer wäßrigen Lösung von gut wasserlöslichen, höhermolekularen, polymerisierbaren Verbindungen, die zwei oder mehr polymerisierbare funktionelle Gruppen pro Molekül und ein Molekulargewicht über 400 besitzen, gemischt werden und die Mischung polymerisiert wird.

2. Verfahren nach Anspruch 1, dadurch gekennzeichnet, daß eine wäßrige Phase, die sowohl das biologische Material als auch höhermolekulare, polymerisierbare Verbindungen enthält, in einer zweiten, nichtwäßrigen Inertphase zu Perlen dispergiert und diese perlförmige Wasserphase polymerisiert wird.

3. Verfahren nach Anspruch 1 und 2, dadurch gekennzeichnet, daß eine wäßrige Lösung oder Dispersion eines biologischen Materials mit einer wäßrigen Lösung von gut wasserlöslichen, höhermolekularen, polymerisierbaren Verbindungen, die zwei oder mehr polymerisierbare funktionelle Gruppen pro Molekül und ein Molekulargewicht von über 400 besitzen, gemischt wird und diese Mischung in einem flüssigen inerten Medium zu Perlen zerteilt und polymerisiert wird.

Le A 22 947

4. Verfahren nach Anspruch 1 bis 3,dadurch gekennzeichnet, daß als Inertphase insbesondere mit Wasser nicht mischbare Stoffe wie aliphatische, cycloaliphatische und aromatische Kohlenwasserstoffe sowie Derivate derselben, Siliconöle, Paraffinöle bzw. auch Gemische derselben untereinander, ver wendet werden.

5. Verfahren nach Anspruch 1 bis 4 ,dadurch gekennzeichnet, daß Perlen der mittleren Durchmesser 0,05 mm bis 5 mm, bevorzugt 0,2 mm bis 2 mm, besonders bevorzugt 0,5 mm bis 1,5 mm demgemäß hergestellt werden.

6. Verfahren nach Anspruch 1 bis 5, dadurch gekennzeichnet, daß das Gewichtsverhältnis Wasserphase zu Inertphase zwischen 1:1 und 1:20 liegt.

7. Verfahren nach Anspruch 1 bis 6, dadurch gekennzeichnet, daß die Zerteilung der wäßrigen Phase zu Perlen durch Rühren und/oder durch Durchleiten von Inertgas oder durch Einsprühen der wäßrigen Phase in die Inertphase erfolgt.

8. Verfahren nach Anspruch 1 bis 7, dadurch gekennzeichnet, daß ein radikalischer Polymerisationsinitiator und/oder ein radikalisches Polymerisationsinitiatorsystem und/oder ein Beschleuniger zugesetzt wird.

Le A 22 947

9. Verfahren nach Anspruch 1 bis 8, dadurch gekennzeichnet, daß ein wasserlöslicher radikalischer Polymerisationsinitiator, insbesondere Ammoniumperoxodisulfat und ein wasserlöslicher Beschleuniger wie N,N,N',N'-Tetramethylethylendiamin zugesetzt wird.

10. Verfahren nach Anspruch 1 bis 9, dadurch gekennzeichnet, daß ein Photoinitiator zugesetzt wird und die Polymerisation durch Bestrahlung erfolgt.

11. Verfahren nach Anspruch 1 bis 10, dadurch gekennzeichnet, daß die Polymerisation durch Bestrahlung mit aktinischem Licht unter Zusatz von in der Inertphase schlechtlösliche oder unlöslichen Photosensibilisatoren erfolgt.

12. Verfahren nach Anspruch 1 bis 11, dadurch gekennzeichnet, daß das Gewischtsverhältnis der wäßrigen Lösung oder der Dispersion des biologischen Materials zu der gut wasserlöslichen polymerisierbaren höhermolekularen Verbindung bzw. deren wäßrigen Lösungen zwischen 1:1 bis 30:1, insbesondere zwischen 4:1 bis 20:1 liegt.

13. Verfahren nach Anspruch 1 bis 12, dadurch gekennzeichnet, daß die gut wasserlöslichen, höhermolekularen, polymerisierbaren Verbindungen aus Polyetherpolyolen, deren Hydroxylgruppen teilweise mit ungesättigten Carbonsäuren verestert und zum anderen Teil mit isocyanatgruppenhaltigen Derivaten ungesättigter Carbonsäuren umgesetzt sind, hergestellt sind.

Le A 22 947

14. Verfahren nach Anspruch 13, dadurch gekennzeichnet, daß

a) die Polyetherpolyole Polyethylenglykole mit einem MW von 400 und größer sind,

b) die ungesättigten Carbonsäuren Acrylsäure und/oder Methacrylsäure sind,

c) die di- oder mehrfunktionellen Isocyanate Isophorondiisocyanat, Toluylendiisocyanat, Hexamethylendiisocyanat, biuretgruppenhaltige Polyisocyanate und/oder Polyisocyanate, die aus der Umsetzung von Diisocyanaten mit mehrwertigen Alkoholen entstehen, sind.

15. Verfahren nach Ansprüche 1-12, dadurch gekennzeichnet, daß die gut wasserlösliche, höhermolekularen, polymerisierbaren Verbindungen aus Polyetherpolyolen, deren Hydroxylgruppen teilweise mit ungesättigten Carbonsäuren verestert und zum anderen Teil mit di- oder mehrfunktionellen Isocyanaten umgesetzt sind, hergestellt sind.

16. Verfahren nach Anspruch 15, dadurch gekennzeichnet, daß

a) die Polyetherpolyole Polyethylenglykole mit einem MW von 400 und größer sind,

b) die ungesättigten Carbonsäuren Acrylsäure und/oder Methacrylsäure sind,

c) die di- oder mehrfunktionellen Isocyanate Isophorondiisocyanat, Toluylendiisocyanat, Hexamethylendiisocyanat, biuretgruppenhaltige Polyisocyanate und/oder Polyisocyanate, die aus der Umsetzung von Diisocyanaten mit mehrwertigen Alkoholen entstehen, sind.

Le A 22 947

17. Verfahren nach einem der vorhergehenden Ansprüche, dadurch gekennzeichnet, daß zur Immobilisierung direkt Fermentationsbrühen, gegebenenfalls nach vorhergehender Konzentrierung, verwendet werden.

18. Verfahren nach einem der vorhergehenden Ansprüche, dadurch gekennzeichnet, daß Zellen oder Enzyme in Gegenwart von Desinfektionsmitteln, Bakteriziden oder Fungiziden immobilisiert werden, somit chemisch sterilisiert in den Bioreaktor überführt werden und die Sterilisationsmittel vor Anlauf der Reaktion aus dem geschlossenen, sterilen Reaktorsystem ausgewaschen werden.

19. Immobilisiertes biologisches Material erhältlich nach einem der vorhergehenden Ansprüche.

20. Immobilisierte Protaminobacter rubrum Zellen erhältlich nach einem der vorhergehenden Ansprüche.

21. Verwendung des immobilisierten biologischen Materials nach einem der vorgehenden Ansprüche zu Biotransformationen.

Le A 22 947